# EUROPEAN PATENT SPECIFICATION

(11) **EP 4 608 937 B1**
(45) Date of publication and mention of the grant of the patent: **03.12.2025**
(21) Application number: 23798736.7
(22) Date of filing: 27.10.2023
(51) Int. Cl.: C10G 2/00, C01B 3/34, C10K 3/02, C25B 1/23

(54) **METHOD FOR PRODUCING HIGHER HYDROCARBONS WITH LOW CARBON FOOTPRINT**
VERFAHREN ZUR HERSTELLUNG HÖHERER KOHLENWASSERSTOFFE MIT NIEDRIGEM KOHLENSTOFFFUSSABDRUCK
PROCÉDÉ DE PRODUCTION D'HYDROCARBURES SUPÉRIEURS À FAIBLE EMPREINTE CARBONE

(30) Priority: 28.10.2022 LU 502973
(43) Date of publication of application: 03.09.2025
(73) Proprietor: Paul Wurth S.A., 1122 Luxembourg (LU)
(72) Inventor: AGRAWAL, Anand Kumar, 3394 Roeser (LU); PIEZANOWSKI, Ludivine, 4067 Esch-sur-Alzette (LU); BOTH, Ingo, 54338 Schweich (DE); DELIKONSTANTIS, Evangelos, 2146 Luxembourg (LU); KINZEL, Klaus Peter, 5222 Sandweiler (LU)
(74) Representative: Office Freylinger
(86) International application number: PCT/EP2023/080080
(87) International publication number: WO 2024/089249

(56) References cited:
- WO-A1-2021/201190
- WO-A1-2022/171643
- US-A1- 2023 111 972
- TAN ZHONGXIN ET AL: "CO2 reforming of biogas to obtain synthesis gas using non-thermal plasma", vol. 90, no. 6, 1 December 2017 (2017-12-01), pages 864 - 874, XP093047242, ISSN: 1743-9671, Retrieved from the Internet <URL:https://www.sciencedirect.com/science/article/pii/S1743967116301805/pdfft?md5=96f0c6064c44e3c44fa64094f4d242d9&pid=1-s2.0-S1743967116301805-main.pdf> DOI: 10.1016/j.joei.2016.08.008
- BLOM P W E ET AL: "Non-catalytic plasma-arc reforming of natural gas with carbon dioxide as the oxidizing agent for the production of synthesis gas or hydrogen", INTERNATIONAL JOURNAL OF HYDROGEN ENERGY, ELSEVIER, AMSTERDAM, NL, vol. 38, no. 14, 1 April 2013 (2013-04-01), pages 5684 - 5692, XP028586726, ISSN: 0360-3199, DOI: 10.1016/J.IJHYDENE.2013.03.042

## Description

### Technical field

The present invention generally relates to the field of, power to liquid or gas to liquid, e-fuel plant applications, such as the production of higher hydrocarbons e.g. for production of synthetic liquid fuels.

### Background Art

Reduction of greenhouse emission, particularly CO₂ emission in the transportation sector is one of the biggest challenges nowadays. While there exist alternatives to fuel consumption for short-distance road transportation sector, there still exist some sectors, such as long-distance road transport, vessel and aviation, where liquid fuel is still largely needed without working substitutes, and thus developing alternative ways of fuel production with lower carbon footprint is of great interest.

The Fisher Tropsch (FT) reaction is a well-known process to produce higher hydrocarbons from CO and H₂, also called syngas, in the presence of a catalyst. The syngas can generally be obtained from biomass, methane or CO₂ valorization, through steam reforming, partial oxidation, reverse water gas shift (RWGS), co-electrolysis, auto-thermal reforming reactions for example. Among all of the syngas production processes known in the art, some of them are non-neutral in terms of carbon footprint while others need further purifications steps. In fact, depending on the feedstock and the reaction conditions, every syngas synthesis pathway will lead to a different composition of syngas, in terms of H₂/CO ratio and in terms of other components or by-products that can be formed during the reaction. However, the FT product is sensitive to the composition of the syngas. For example, generally when the syngas is produced by gasification of the biomass, a step of purification of the syngas is needed before using it in the FT reaction.

WO2021/201190 and WO2022/171643 both disclose methods for producing hydrocarbons from carbon dioxide and hydrogen. The methods both use electrolysis, reverse water gas shift and Fischer-Tropsch.

### Technical problem

It is an object of the present invention to produce high quality and valuable higher hydrocarbons with a reduced carbon footprint. Such higher hydrocarbons can be advantageously used as fuels in sectors, such as the aviation and transportation sector. The object of the invention would thus be to propose an improved, efficient and sustainable FT process, optimized to produce a valuable product, preferably a valuable liquid fuel, the FT process aiming at an overall reduction of the carbon footprint.

### General Description of the Invention

To achieve this object, the present invention proposes a method in accordance with claim 1.

The FT process is converting syngas (CO + H₂) into higher hydrocarbons and other (undesired) components, such as lower or gaseous hydrocarbons and water. When CO₂ and H₂O are the key resources for the production of liquid fuel in the FT reaction, the process is called syngas to liquid process. If the renewable electrical energy is used for the production of syngas via this route then the process is also referred as e-fuel production process. For an e-fuel plant several possibilities of utilizing electrical energy for the production of syngas may exists. For example, CO₂ can be obtained via direct air capture through electrical energy, H₂ can be produced via electrolysis, syngas can be directly produced by co-electrolysis or by the reverse water gas shift reaction utilizing electrical energy.

The FT reactor generally operates in the presence of a catalyst to convert the syngas. During the FT reaction, a mixture of liquid hydrocarbons is formed and separated as FT synthetic crude, while the mixture of lower hydrocarbons (< C6) and other gaseous components such as H₂O, CO₂, N₂ that are inert to FT reaction, as well as unreacted CO and H₂, forms the gaseous phase and is often referred to as FT tail-gas. The mixture of higher hydrocarbons fraction of the FT reaction is generally upgraded in the upgrading unit or product workup unit including a final step of distillation to give the desired liquid fuel(s).

According to the present invention, higher hydrocarbons refer to the fraction of hydrocarbons produced during the FT reaction having a number of carbon atoms higher than 6. In other terms, higher hydrocarbons refer to hydrocarbons having a long chain, in the range of C6 to C100, and wherein the higher hydrocarbons can be liquid at normal temperature and pressure conditions (NTP, 20 °C, 1 atm) and/or can be solid, such as waxes. During the FT reaction, non-condensable fractions or lower fractions (with a carbon chain with up to 5 carbon atoms) are also produced, and are referred in the present invention as lower hydrocarbons or lower fractions. Other gases or unreacted gases, such as CO₂, N₂, oxygenates and unreacted syngas (CO, H₂), is referring herein to as FT tail-gas. Furthermore, "dry syngas" refers to a syngas comprising < 5 vol.-% of H₂O, preferably < about 3 vol.-%, more preferably about < 1 vol.% of H₂O.

The FT products are normally separated into two streams, the higher hydrocarbons streams that are ongoing for further treatment such as separation and/or upgrading, and the lower (gaseous) fractions stream, i.e. said FT tail-gas.

Downstream the FT unit, upgrading and distillation units are generally installed in order to produce, from the higher hydrocarbons stream, desired fraction(s) of hydrocarbons, wherein the said fraction(s) can be fuels such as kerosene, naphtha, diesel, gasoline, etc.

Conventionally, the syngas can be obtained by different reforming reactions. Advantageously, the syngas can be obtained from a source of CO₂. In that case, basically two main routes are available to convert CO₂ into syngas. One of them is the reverse water gas shift reaction (RWGS) that converts CO₂ into CO and water in the presence of a large excess of H₂. The RWGS reaction is the reverse process of the chemical equilibrium Water Gas Shift reaction where CO₂ and H₂ are produced from CO and H₂O. With the idea to reduce the carbon footprint of industrial process and to make use of the anthropic CO₂, the WGS reaction can be pushed towards the consumption of CO₂ to form the CO, this reaction is called RWGS reaction. Large excess of H₂ is preferable to reverse the WGS reaction.

Alternatively, CO₂ and H₂O can be converted to syngas (CO + H₂) via co-electrolysis. As it is known to those skilled in the art, the co-electrolysis (also co-electrolysis process) occurs in a co-electrolyser reactor, in the presence of streams of CO₂ and H₂O that are electrolyzed. The FT tail-gas can also be treated in the co-electrolyser combined with classical catalyst-based reformer to form a syngas.

The conversion of CO₂ into syngas via the RWGS reaction or via the co-electrolysis can indeed represent a viable route to convert CO₂ into CO. Since the CO₂ is already available in the atmosphere or is emitted through various industrial processes and transportation, using this CO₂ represent a good way to reduce greenhouse emissions by transforming the CO₂ into a valuable gas for the production of liquid fuel and thus reducing the overall carbon footprint of the process itself and processes downstream using said fuels.

According to the invention plasma-based treatment refers to reactions occurring at a plasma state of matter in a plasma-based reactor. In the plasma-based reactor, several reactions can take place, such as but not limited to, steam methane reforming, dry reforming, partial oxidation, auto-thermal reforming and reverse water gas shift reaction allowing to transform a maximum amount of gases entering the plasma-based reactor into syngas depending on the reactants present. In embodiments, the plasma-based treatment is effected in a plasma-based reactor where syngas is produced mainly from streams of FT tail-gas and optionally a regulating agent. Regulating agent according to the present invention, is an additional stream of gases that can be injected in the plasma-based reactor to control or drive the formation of the syngas and the composition of the syngas formed in the said plasma-based reactor. The regulating agent can be, CO₂, H₂, H₂O, O₂ or a mixture of them.

In further embodiments, the plasma-based treatment is integrated into a unit where inlet of CO₂ and H₂ are provided to allow the RWGS reaction to occur in a plasma-based reactor, a so-called plasma-based RWGS reactor. In the plasma-based RWGS reactor, among the reactions that takes place in the plasma-based reactor such as defined above, the main reaction occurring is therefore the RWGS reaction due to further addition of CO₂ and H₂. Therefore, in the plasma-based RWGS reactor, syngas is produced mainly from streams of CO₂, H₂ and FT tail-gas. Moreover, an optional regulating agent can be added in the plasma-based RWGS reactor, if a further control of the syngas composition is desired or needed.

In the present context, the expression "hot syngas" refers to the syngas formed during a reforming process, i.e. RWGS reaction and/or co-electrolysis and/or plasma-based treatment. In other terms, hot syngas refers to syngas exiting the RWGS reactor, and/or the co-electrolyser reactor and/or the plasma-based reactor and/or the plasma-based RWGS reactor. The expression "cooled syngas" refers to the syngas after treatment in a cooling step. The expression "dry syngas" refers to the syngas that is sent to the FT reactor. Unless otherwise indicated, when the term "syngas" is used alone in the present document, it may refer to the hot syngas, and/or the cooled syngas, and/or the dry syngas.

The FT reaction produces higher hydrocarbons, such as a crude liquid fuel, that can be upgraded in an upgrading/distillation process to obtain defined fractions or pure higher hydrocarbons as synthetic fuel with the desired carbon chain composition, density, and/or viscosity for example. Advantageously, the higher hydrocarbons from the FT reactor are further fed to a downstream separation step, such as in a distillation unit, to obtain one or more defined fractions of higher hydrocarbons and a separation/distillation waste fraction. The separation/distillation waste fraction is advantageously also be treated by said plasma-based treatment, in the plasma-based reactor and/or in the plasma-based RWGS reactor. The distillation waste fraction is commonly composed of undesired components recovered during the upgrading/distillation step, and is advantageously also recycled back to the plasma-based reactor to undergo the plasma-based treatment.

The one or more defined fractions of higher hydrocarbons, also called defined fractions, refer to the desired compounds that are recovered after the separation process, such as after distillation or upgrading. The defined fractions generally represent about 65 vol.-%, preferably about higher than 75 vol.-%, of the higher hydrocarbons fraction before separation/distillation, and the proportion depends, among others, on the FT reaction conditions. Among the defined fractions, gasoline, kerosene and/or diesel are particularly preferred. Further defined fractions may also be e.g. paraffins/waxes or other oxygenated hydrocarbon fractions.

In embodiments, the production within the FT reactor may advantageously be controlled to more specifically/predominantly produce certain defined fractions. One way to control the reaction within the FT reactor is to control the composition of the dry syngas entering the FT unit. As already mentioned above, the expression "dry syngas" as used herein means the syngas that enters the FT reactor and generally represents a syngas comprising very low amount of water, i.e. < about 5 vol.-%, preferably < about 3 vol.-%, more preferably about < 1 vol.% of H₂O. In embodiments, wherein fuel fractions, such as gasoline, kerosene and/or diesel are preferred, the dry syngas may have a H₂/CO ratio of about 1.70/1 to about 2.50/1, preferably of about 1.80/1 to about 2.30/1, more preferably about 1.90/1 to about 2.10/1. The composition of the dry syngas and more particularly the H₂/CO ratio may be controlled e.g. by adding regulating streams comprising CO₂, and/or H₂, and/or H₂O and/or O₂ to the plasma-based treatment of FT tail gas, and/or e.g. by controlling the stream of CO₂ and/or the stream of H₂ fed to the RWGS reaction, and/or e.g. by controlling the stream of CO₂ and/or H₂O and/or FT tail-gas fed to the co-electrolyser, and/or e.g. by mixing an additional stream of H₂ to the hot, cooled or dry syngas. The H₂/CO ratio herein refers to the molar ratio of H₂ to that of CO in the dry syngas. Since the composition of the higher hydrocarbons produced by the FT reaction, depends on the H₂/CO ratio of the syngas, monitoring and controlling this ratio by means of regulating streams positioned at different locations of the fuel plant, is a great advantage. In embodiments, the ratio can be controlled by injecting regulating stream in the plasma-based treatment, thus controlling the plasma-based treatment reaction depending on the composition of the gas entering the plasma-based treatment. This control, allows to further optimize the reforming reaction condition, to favorably transform the undesired components into syngas and thus to obtain the ideal hot syngas composition.

In the present invention, the dry syngas is partially formed by plasma-based treatment of the FT tail-gas, which advantageously can also comprise the separation/distillation waste fraction. The regulating stream added to the plasma-based treatment of the FT tail-gas allows to better control and optimize the conversion of the FT tail-gas into syngas in the plasma-based treatment, thus forming a syngas comprising mainly CO and H₂ and further components. Further components, as defined herein, are either undesired or inert for FT reactions, and may come from uncomplete reactions or that may be by-products in the RWGS reaction, and/or in the co-electrolyser and/or in the plasma-based treatment. Further components may be e.g. lower hydrocarbons, N₂, CO₂, H₂O etc.

The gas present in the FT tail-gas that are not CO and H₂, can specifically react with the regulating streams to obtain the desired syngas. Moreover, the composition of the regulating streams can be advantageously adjusted depending on the FT tail-gas composition to optimize the conversion. The regulating stream can not only control the FT tail-gas conversion into syngas and thus allow to obtain a minimum concentration of the further components in the syngas, but it also can advantageously control the resulting ratio of H₂/CO in the syngas, e.g. by adding a controlled excess amount of H₂ in the plasma-based treatment.

In the prior art, the FT tail-gas is generally vented to avoid an undesirable build-up of unwanted components. Indeed, when FT tail-gas is continuously reinjected in the FT reaction loop, the inert or undesired components tend to accumulate in the loop, thereby reducing the efficiency of the conversion of syngas into liquid fuel and increasing volumetric flowrates handled within the process. Advantageously, using plasma-based treatment of the FT tail-gas can drastically reduce the proportion of undesired compounds accumulating in the loop, while allowing further control of the FT tail-gas reforming. Having said this, even when FT tail-gas is recycled with a plasma-based treatment as proposed herein, it might nevertheless become necessary at times to vent undesired gas or to reused elsewhere, such as fuel gas. Indeed, in the plasma-based treatment, different reactions can occur, such as, but not limited to, steam methane reforming, dry reforming, partial oxidation, auto-thermal reforming and reverse water gas shift reaction allowing to transform a maximum amount of gases entering the plasma-based treatment into syngas, therefore, the FT tail-gas entering the plasma-based reactor can advantageously be transformed into hot syngas, and only small amounts of FT tail-gas is not transformed and is still present in the hot syngas.

According to the invention, part of the dry syngas going to the FT reactor is formed by RWGS reaction, that converts CO₂ and H₂ into CO and H₂O and/or by co-electrolysis, that converts CO₂ and H₂O into CO and H₂. The control of dry syngas composition can be done at different stages of the dry syngas formation. It is possible to control the dry syngas composition by adjusting the inlet of CO₂ and H₂O when the dry syngas is produced by co-electrolysis. It is also possible to control and adjust the composition of the dry syngas by controlling the streams of CO₂ and/or H₂ entering the RWGS reaction to adjust the molar H₂/CO ratio. RWGS reaction transforms CO₂ and H₂ into CO and H₂O, and the reaction equilibrium can be shifted to the high conversion into CO by adding an excess of H₂. Advantageously, the excess of H₂ that did not react can be used to optimize and more finely tune the H₂/CO ratio of the resulting hot syngas. If H₂ is added upstream a RWGS reactor, it needs to be heated prior injection, to pass through the RWGS reactor, or in the reactor itself. In embodiments, a stream of H₂ is mixed with the stream of hot syngas, and/or is mixed with the stream of cooled syngas and/or is mixed with the stream of dry syngas.

Advantageously, when H₂ is added in the cooled syngas and/or the dry syngas, i.e. downstream the cooling step, H₂ does not need to be heated first, therefore resulting in saving heating energy. Moreover, H₂ can also be introduced in the hot syngas exiting the plasma-based reactor, and/or the plasma-based RWGS reactor, and/or the RWGS reactor. As plasma-based treatment and RWGS reaction occurs at high temperature, higher than about 1250° C, the H₂ added in the hot syngas stream, will cool said hot syngas stream at the desired temperature and thus also quench the reforming reaction to obtain the desired syngas composition and temperature. This will permit to reduce the energy consumption of the overall process, since the H₂ will serve as a cooler and thus save energy losses that might occur during cooling. In other terms, when the H₂ stream is mixed with the hot syngas downstream the plasma-based reactor, there is not only no need to heat it at high temperature beforehand, but the H₂ added downstream may also be used to quench the hot syngas generated at the plasma-based reactor, both of which are highly beneficial in terms of energy saving.

The composition of the resulting dry syngas entering the FT reactor will influence the performance of the FT reaction and/or the composition of the resulting hydrocarbons, therefore controlling and adjusting the gas composition with regulating streams is a simple, yet powerful feature and allows to obtain an optimized dry syngas mixture for the FT reaction depending on the desired defined fractions.

Advantageously, further components (as defined above) within the dry syngas, i.e. the syngas entering the FT reactor, represent < about 50 vol.-%, preferably < about 35 vol.-%, such as < about 30 vol.-%, more preferably < about 25 vol.-%, such as < about 20 vol.-%, even more preferably < about 15 vol.-%, of dry syngas. Water can be an inhibitor of the reaction, and CO₂ as well as lower hydrocarbons such as methane, and other gas are inert to the FT reaction. A further advantage is that by controlling the total concentration of such components entering the FT reactor, the FT reaction can be still further optimized. Such components could be removed from the FT tail-gas, when is it recycled. In the present invention however, the FT tail-gas is treated by a high-temperature plasma which allows for a more efficient recycling of FT tail-gas into the process, and the plasma-based treatment is further optimizable by the addition of the above-mentioned regulating stream, to obtain the dry syngas suitable for the desired operation of the FT reaction. These parameters will greatly influence the carbon conversion yield of the overall process and will reduce CO₂ and other waste gas emission, overall being highly beneficial in terms of carbon footprint.

As the FT reaction and the resulting FT products depend on the H₂/CO ratio, the FT conversion of syngas into higher hydrocarbons is also influenced by the presence of further components in the dry syngas, that can be either inhibitors of the reaction or inert to the reaction. As H₂O is a reaction product of the FT reaction, it will generally act as an inhibitor of the FT reaction. Therefore, reducing its amount in the dry syngas is advantageous. Hence, a dry syngas as defined herein preferably means that this syngas has a concentration of H₂O < about 5 vol.-%, preferably < about 3 vol.-%, more preferably about <1 vol.% of H₂O.

The FT reaction can operate at temperatures between 330 - 370° C, generally referred to as High Temperature FT (HTFT). HTFT provides products in the naphtha range containing linear and branched olefins with high aromatics and oxygenate contents. When FT operates at temperatures between 210 - 260° C, the process is referred to as Low Temperature FT (LTFT), and the syngas is converted into linear long-chained paraffins and lighter olefins, methane, petroleum gas, naphtha, kerosene and waxes. In embodiments, the FT reactor can be either HTFT or LTFT, so generally it can be operated at temperatures from about 180 °C to about 350 °C, preferably from about 200 °C to about 230 °C. Further, the FT reactor is advantageously operated at pressures from about 5 to about 60 barg, preferably from about 10 to about 50 barg, more preferably from about 20 to about 45 barg, even more preferably between 20 to 30 barg. Still further, the FT reactor preferably makes use of a catalyst, such as e.g. one or more cobalt and/or iron catalyst compounds. The FT reaction conditions are preferably optimized to (preferentially) obtain the desired defined fraction(s). When the FT reactor operates at the lowest temperature, between about 180 °C to about 200 °C, longer hydrocarbons chains can generally be obtained such as paraffins and/or olefins derivatives. At preferred temperatures from about 200 °C to about 230 °C, the mainly higher hydrocarbons produced can advantageously be kerosene, diesel or other higher hydrocarbons after upgrading step. The selectivity of the FT synthesis can also be controlled by the choice of the catalyst.

In embodiments, the stream of H₂ is produced by electrolysis and/or the stream of CO₂ is recovered from direct air capture, combustion gases and/or other industrial off-gases, preferably from direct air capture. The process consumes CO₂, which can be obtained as a product or byproducts from various industrial processes and preferably from sources such as biomass or direct air capture, thus drastically reducing the carbon footprint of the process and thus of the hydrocarbon products. One advantage of using CO₂ as the starting material of the liquid fuel production, is that CO₂ is an important and main greenhouse gas, mainly emitted from oil refineries, power plants, steel production, cement plant and transportation, and can now be reused as a valuable starting material for the production of liquid fuel in an overall almost neutral carbon footprint. Therefore, the CO₂ is not considered as a waste, but rather as a valuable carbon source for the production of liquid fuel. The higher hydrocarbon obtained by this process can be used as fuel for transportation, aviation for example. The recycling of such CO₂ emission can lead to a greener liquid fuel production and utilization and to a drastic decrease of CO₂ in the atmosphere, even can lead to a neutral carbon footprint, thus can help to reach the Paris agreement in terms on greenhouse gas emission.

The control and optimization of the plasma-based treatment can be an advantage in the optimization of the FT tail-gas recycling. In embodiments, the plasma-based treatment is promoted by plasma at temperatures higher than about 1000 °C, preferably higher than about 1250 °C, more preferably higher than about 1500 °C, even more preferably higher than about 2000 °C, such as higher than about 2500 °C, higher than about 3000 °C or higher than about 4000 °C, even up to or more than 5000 °C and/or at pressures lower than about 45 barg, such as lower than 40 or 30 barg, preferably lower than about 20 or 15 barg, more preferably lower than 10 barg, such as lower than 7.5 barg, even more preferably lower than 5 barg. The plasma-based treatment unit may include any appropriate means for generating a plasma for effecting the plasma-based treatment. In embodiments, the plasma-based treatment unit may comprise one or more plasma torches, preferably electrode-based plasma torches and/or electrodeless plasma torches that ignite transferred and/or non-transferred arcs. The one or more plasma torches may include direct current plasma torches, alternating current plasma torches, 3-phase alternating current plasma torches or a combination thereof. The said plasma torches may have a total electric power of about 1 to about 20 MW, preferably of about 3 to about 15 MW, more preferably of about 5 to about 10 MW. The electrodeless plasma torches may include inductively ignited plasma torches, microwave plasma torches, radiofrequency plasma torches or a combination thereof.

In view of reducing CO₂ emissions and more generally, the emission of gas produced during industrial process, a fine control of the syngas production in the plasma-based treatment is an advantage. One advantage of the plasma-based treatment is that it allows high conversion rates of CO₂ compared to conventional thermal/thermocatalytic processes such as reverse water gas shift and dry reforming hydrocarbons with CO₂. The CO₂ referred here can be the CO₂ present in the FT tail-gas, or the CO₂ from the regulating stream. Another advantage of the plasma-based treatment, is the conversion of the further components present in the FT tail-gas into syngas without the need of a catalyst. In the plasma-based treatment, several reforming reactions can occur, such as, but not limited to steam methane reforming, dry reforming of hydrocarbons with CO₂, partial oxidation, auto-thermal reforming and (reverse) water gas shift reforming, leading to higher rate of conversion, and lower further components concentration in the resulting syngas. In embodiments the FT tail-gas can be fully recycled, in other embodiments the FT tail-gas can be partially recycled and partially vented or utilized elsewhere.

In embodiments, the invention also describes a method wherein the RWGS reaction is effected in a RWGS reactor to produce a first stream of hot syngas, the first stream of hot syngas being treated in a first quenching/cooling/heat recovery unit to form a first stream of cooled syngas; and wherein, the plasma-based treatment of FT tail-gas is effected in a plasma-based reactor to produce a second stream of hot syngas, the second stream of hot syngas being treated in a second quenching/cooling/heat recovery unit to form a second stream of cooled syngas; and wherein the first and second streams of cooled syngas are dried, if needed compressed, and mixed in a drying//mixing unit to obtain the dry syngas. Advantageously, the desired H₂/CO ratio of the resulting second stream of hot syngas is fixed in the quenching/cooling/heat recovery unit, thus avoiding a reverse reaction that can take place at low temperature, during the cooling step, and that can lead to a lower syngas quality for the FT reaction, forming the second stream of cooled syngas. The quenching/cooling/heat recovery step may involve a quenching/cooling step where the hot syngas is cooled down to a temperature between about 400 to about 500 °C, a further heat recovery step, e.g. via a heat exchanger, by which syngas heat is transferred to another fluid/stream to allow the syngas to reach a temperature preferably between about 20 °C to about 80 °C.

The first stream of hot syngas from the RWGS reactor, is also quenched and cooled to obtain a first stream of cooled syngas that is advantageously mixed with the second stream of cooled syngas from the plasma-based treatment. The RWGS reaction is effected in a RWGS reactor operating at temperature between about 750 °C to about 1200 °C, preferably between about 800 °C to about 1000 °C, more preferably between 850 °C to about 950 °C, at a pressure between about 1 to about 40 bar, preferably between about 3 to about 30 bar, more preferably between about 5 to about 15 bar, and/or in the presence of a catalyst. In embodiments, when exiting the plasma-based treatment, the second stream of hot syngas has a temperature higher than 900 °C to about 2000 °C or even higher than about 2000 °C, the second stream of cooled syngas has advantageously a temperature lower than about 500 °C, preferably between about 400 °C to about 500 °C, more preferably lower than about 400 °C. In embodiments, when exiting the RWGS reactor, the first stream of hot syngas has a temperature from about 800 °C to about 1500 °C, preferably from about 900 to 1200 C, more preferably from about 800 to about 1100° C, even more preferably from about 800° C to about 950° C, the first stream of cooled syngas has advantageously a temperature lower than about 500 °C, preferably lower than about 400 °C, more preferably lower than about 80 °C. A regulating stream of H₂ can be added in the first and/or second stream of hot syngas, to quench the reaction and fix the syngas composition. The resulting cooled syngas is preferably dried to remove water formed during the previous reaction, leading to dry syngas suitable for FT reaction. At this step, a further stream of H₂ can be advantageously added to adjust the final ratio of H₂/CO of the resulting dry syngas before entering the FT reactor. Another advantage of the sequential cooling/drying steps, is the control of the temperature of the resulting dry syngas, that will enter the FT reactor at the defined reaction temperature. Another advantage, is that the steam formed during the cooling/drying step can be further used as a source of H₂O for the electrolysis production of H₂, thus recycling and minimizing the net H₂O needed for the process by closing the loop of H₂ production, which is an advantage in terms of sustainability of the process.

In embodiments, the invention describes a method wherein the co-electrolysis is done in a co-electrolyser reactor to produce a first stream of hot syngas, the first stream of hot syngas being treated in a first quenching/cooling/heat recovery unit to form a first stream of cooled syngas; and wherein, the plasma-based treatment of FT tail-gas is done in a plasma-based reactor to produce a second stream of hot syngas, the second stream of hot syngas being treated in a second quenching/cooling/heat recovery unit to form a second stream of cooled syngas; and wherein the first and second streams of cooled syngas are dried and preferably mixed in a drying/compression/mixing unit to obtain the dry syngas. Advantageously, a stream of H₂ can be added to the second stream of hot syngas to quench the reaction and fix the syngas composition. A stream of H₂ can also be mixed with the first stream of cooled syngas or mixed with the dry syngas.

In other embodiments, the invention describes a method wherein the RWGS reaction and the plasma-based treatment are effected in a plasma-based RWGS reactor to form a combined stream of hot syngas; wherein the combined stream of hot syngas is treated in a combined quenching/cooling/heat recovery unit to obtain the dry syngas. The plasma-based RWGS reactor refers to a reforming unit where RWGS reaction, converting CO₂ into CO, takes place in plasma-based conditions at a temperature higher than about 1250 °C, preferably higher than about 1500 °C or still higher as mentioned above. In this condition the conversion of CO₂ is optimized and the inlet of H₂ is reduced. The RWGS reaction needs high excess of H₂ to efficiently convert CO₂. The high temperature in the plasma-based RWGS reactor allows the conversion of CO₂ in high conversion yield without the need of adding an excess of H₂. This reactions condition offers the possibility to convert CO₂ into CO in a good conversion yield and to reduce H₂ consumption, thus reduce H₂ production, reducing the overall energy needed to produce the final liquid fuel. Another advantage of the high temperature condition and the plasma-based RWGS reactor, is that the reaction takes place well above the thermodynamic equilibrium for conversion of CO₂ into syngas, advantageously reducing the excess H₂ inlet rendering the catalyst use unnecessary for the RWGS reaction. Moreover, the FT tail-gas that is recycled into the plasma-based RWGS reactor also reduces the concentration of H₂ that needs to be added to perform the RWGS reaction, thus reducing the power consumption demand when H₂ is produced on site. The reduction in production and utilization of H₂ is again an advantage in terms of sustainability of the overall process. Another advantage of using plasma-based treatment as reforming unit, is the high conversion rate of hydrocarbons and other gas, further components, from FT tail-gas and distillation waste gases into syngas, thus allowing to recycle it back in the FT loop. The almost full conversion of the further components, e.g.CO₂, lower hydrocarbons, reduces the concentration of the said further components that can accumulate during the recycling loop, and thus the amount of vented gases is reduced compared to conventional reforming FT tail-gas recycling loop.

In embodiments, a stream of H₂ is added in the combined stream of hot syngas, to quench and fix the syngas composition, i.e. H₂/CO ratio.

In other embodiments, the invention describes a method wherein the plasma-based treatment of FT tail-gas is effected in a plasma-based reactor to produce a second stream of hot syngas, the second stream of hot syngas being mixed with streams of CO₂ and H₂ to form a third stream of hot syngas; and wherein the third stream of hot gas is treated in a RWGS reactor to form a fourth stream of hot syngas; and wherein the fourth stream of hot syngas is cooled and dried in a quenching/cooling/heat recovery/drying unit to form a stream of dry syngas. The second stream of hot syngas exiting the plasma-based reactor has a temperature higher than about 1250 °C, preferably higher than about 1500 °C, more preferably higher than about 1750 °C. This hot syngas is being mixed with CO₂ and H₂ streams prior to being submitted to the RWGS reaction. Depending on the temperature and flow of the second stream of hot syngas, and the desired flow of the third stream of hot syngas, additional heating step can be provided either before or in the RWGS reactor in order to reach the desired temperature needed for the RWGS reaction. Advantageously, no additional heating step is required if during the mixing step of streams of CO₂ and H₂ with the second stream of hot syngas, the resulting gas mixture is heated at a temperature above about 900 °C, preferably above about 950 °C. The third stream of hot syngas is further sent to the RWGS reactor at a relative high temperature, thus limiting the heating energy needed to heat up the gas to perform the RWGS reaction. Advantageously, this will simplify the process and enhance energy efficiency of the overall process. Moreover, this will reduce the carbon footprint as FT tail-gas is efficiently utilized in the process for production of liquid fuel.

Advantageously, a stream of H₂ is added in the stream of dry syngas.

In further embodiments, the streams of hot syngas are compressed after being treated in the quenching/cooling/heat recovery unit before mixing together. Moreover, the dry syngas can also be compressed after drying/mixing and before being treated in the FT reactor. The compression step will take place in a compressor unit, when needed, i.e. when there is a difference of pressure between the streams.

The heat from hot syngas can also advantageously be recovered in a heat recovery unit during cooling. Said recovered heat can be further used to heat the inlet FT tail-gas entering the plasma-based reactor and/or to heat the inlet gas for RWGS reactor and/or to heat the dry syngas prior entering the FT reactor.

The heat generated during quenching can be used for production of steam, that can be further used in the electrolyser unit to produce H₂ and/or syngas.

In further embodiments, the first and/or second stream of hot syngas, and/or the combined stream of hot syngas, and/or the first and/or second stream of cooled syngas, and/or the combined stream of cooled syngas, and/or the stream of dry syngas is compressed in a compression unit, preferably the first and/or second stream of cooled and/or the stream of cooled syngas, more preferably, the dry syngas is compressed in a compression unit. The hot syngas formed at the RWGS reactor and/or the co-electrolyser reactor, and/or the plasma-based reactor and/or the plasma-based RWGS reactor generally have different pressures when exiting their respective reactor. Advantageously, a compression step can be realized to adjust the pressure of the syngas prior to the next step. The compression can be effected in a combined quenching/cooling/heat recovery unit and/or in a separate unit. The compression is preferably performed after the heat recovery step, more preferably after the drying step. However, the compression step can be realized at different stages of the process, that can be choose by the skilled person to optimize the process.

"About" in the present context, means that a given numeric value covers a range of values from -10 % to + 10% of said numeric value, preferably a range of values from -5 % to +5 % of said numeric value or even a range of values from -2.5 % to +2.5 % of said numeric value.

### Brief Description of the Drawings

Preferred embodiments of the invention will now be described, by way of example, with reference to the accompanying drawings in which:
Fig. 1 is a schematic view of a first embodiment;
Fig. 2 is a schematic view of a second embodiment;
Fig. 3 is a schematic view of a third embodiment; and
Fig. 4 is a schematic view of a fourth embodiment.

Further details and advantages of the present invention will be apparent from the following detailed description of several not limiting embodiments with reference to the attached drawings.

### Description of Preferred Embodiments

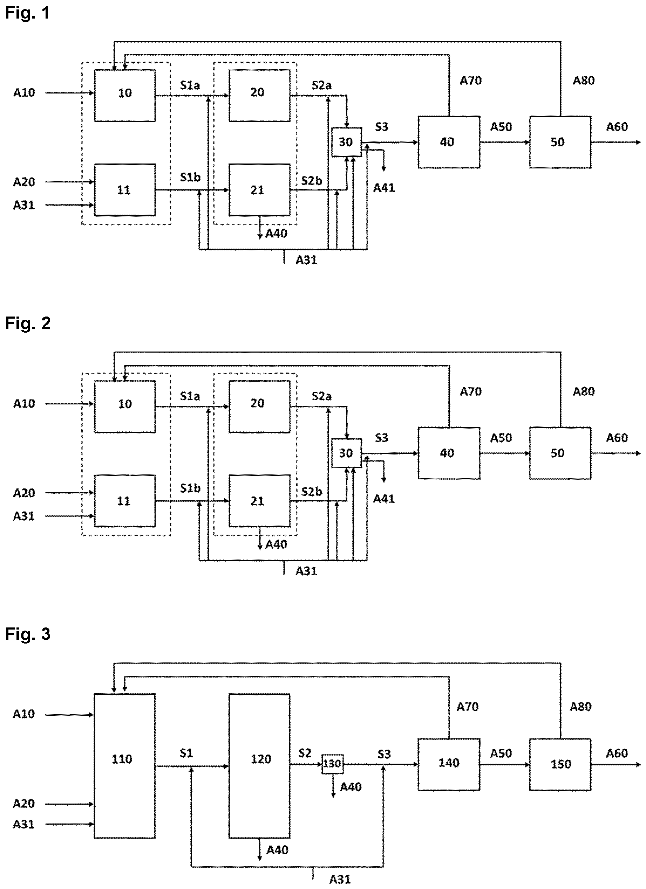

Fig.1 shows a fuel plant installation comprising a Reverse Water-Gas shift reactor RWGS 11 to which H₂ produced e.g. by H₂O electrolysis or other sources, and CO₂ e.g. from Direct Air Capture or from other sources are introduced via streams A20 and A31 respectively. The RWGS reactor operates at a temperature of higher than about 850 °C, and is electrically heated or otherwise heated or electrical heater is coupled with RWGS to heat the feed gases. The first hot Syngas S1b produced at the RWGS reactor 11 contains mainly CO and H₂ gas. Few percentages of H₂O and CO₂ are present as well. The first hot syngas S1b, is further cooled in a quenching/cooling/heat recovery unit 21 to produce the first cooled syngas S2b. Quenching/cooling/heat recovery unit 21 may contain heat recovery unit or setup such as process gas boiler to utilize the sensible heat of the syngas coming from unit 21. This recovered heat can be used to heat up the inlet stream A10 and/or A20 and/or A70 and/or S3 or even for other purposes such as production of steam for the electrolyser or elsewhere.

In parallel, the FT tail-gas A70 exiting the FT reactor 40, is sent in the plasma-based reactor 10 to be transformed into second stream of hot syngas S1a. In a preferred embodiment, the regulation stream (CO₂, H₂, O₂, H₂O, CO, hydrocarbon gas) A10 is injected inside the plasma based-treatment reactor 10 to regulate the concentration of gases, and the final composition of hot syngas S1a. Gas detection probes (not shown) can be positioned upstream and/or downstream the plasma-based treatment reactor 10 to control the gas composition to better tune the final dry syngas composition depending on the gas composition entering the plasma-based treatment reactor 10. Reactor 10 generally operates at a temperature higher than about 1500 °C and a pressure of lower than about 45 barg, preferably lower than about 10 barg, and the resulting hot syngas is further fast cooled/quenched in a quenching/cooling/heat recovery unit 20 avoiding the activation of the reverse reaction during the cooling process. Quenching/cooling/heat recovery unit 20 may contain a heat recovery unit to utilize the sensible heat of the syngas coming from unit 20. This recovered heat can be used to heat up the inlet stream A10 and/or A20 and/or A70 and/or S3 or even for some other purpose such as production of steam for electrolysis or elsewhere.

The resulting cooled syngas streams S2a from unit 20 and S2b from unit 21 are dried and mixed in a drying /mixing unit 30 to provide the dry syngas S3. A compression step (not shown) might be needed either for S2a and/or S2b and even after drying/mixing unit 30 depending on the pressure difference between S2a, S2b and the required pressure for FT reactor unit 40.

In a preferred embodiment, the dry syngas S3, has a gas composition comprising a molar ratio of H₂/CO between 1.90/1 to 2.10/1, and the further components that are inert to FT reaction CO₂, N₂, lower hydrocarbons represent less than 35 vol.-% of the total composition of the dry syngas S3. Moreover, the amount of H₂O is preferably less than 1 vol.-%, to optimize the FT reaction. In order to adjust the final ratio of H₂/CO of dry syngas S3, before being injected to the FT reactor unit 40, an additional stream A31 of H₂ can be injected in the mixing unit 30 and/or in the stream S2b, and/or S2a, and/or S3. Controlling the H₂ content by adding stream A31 directly to the mixing unit 30 and/or to the cooled syngas S2b and/or S2a, and/or dry syngas S3 allows to avoid heating H₂ at high temperature, and thus reduce the energy needed for operating the e-fuel plant. The stream A31 can be used to also control the pressure of the cooled and dry syngas.

The dry syngas stream S3 is further injected in the FT reactor unit 40 that operates at a temperature preferably between 200-230° C, a pressure of 20 - 30 barg, with cobalt or iron as bed catalyst. The resulting stream is divided into streams A50 and A70. Stream A50 containing higher hydrocarbons, i.e. liquid hydrocarbons, waxes, and other oxygenated components is further treated in an upgrading/distillation unit to give the stream A60 as the defined fraction of higher hydrocarbons. The FT tail-gas stream A70, containing unreacted components of dry syngas S3 and further components resulting from the FT reaction such as hydrocarbons from C1-C5, moisture, is sent back to the plasma-based treatment reactor 10 to be recycled. Moreover, the distillation waste gas, stream A80 exiting the upgrading/distillation unit 50, and comprising mainly CO₂, CO, H₂, H₂O, hydrocarbons, N₂, is sent back to the plasma-based treatment reactor 10 to be recycled.

In a preferred embodiment, the stream A60 of defined higher hydrocarbons produced at unit 50 is kerosene, naphtha is formed as a byproduct. In other embodiments, diesel can be recovered, and other components such as paraffin or oxygenated compounds can also be recovered depending on the FT reactor 40 and/or unit 50 parameters.

Additionally, the condensate/steam produced at unit 21, stream A40, can be used as a source for the production of H₂ by electrolysis (not shown).

FT process is converting syngas into hydrocarbons. Usually, heavy hydrocarbons are taken out as waxes and condensates, whereas unreacted syngas (CO + H₂) and components that are inert to FT process such as lower hydrocarbons (< C5), CO₂, N₂, leaves the FT process with the waste gas also known as FT tail-gas A70. If FT tail-gas is recycled back to the FT process, H₂ and CO take part of the FT reaction, while hydrocarbons as well as CO₂ are inert component for FT, accumulates over the time. Therefore, it is necessary to eliminate it from the loop either by directly venting FT tail-gas to atmosphere or utilizing it as a fuel anywhere in the industrial plant. However, venting FT tail-gas or utilizing it elsewhere, as a fuel decreases the product and carbon yield as well as the energy efficiency of the e-fuel plant. Same is applicable for distillation waste gases.

In the present process, however, FT tail gas and distillation wastes gases are sent to the plasma-based reactor 10 to be transformed into syngas that can be further recycled in the FT process. The plasma-based treatment allows to transform the majority of hydrocarbons, CO₂, H₂O contained in the FT tail-gas and distillation waste gas, into syngas, thus reducing significantly the further components that are sent back to the FT reactor without the need to vent the further components at each loop. However, some further components may not be transformed into syngas, and may accumulate during the loop, therefore, optionally, the further components might be vented (not shown) when required. Nevertheless, by using feed gas A20, A30 and A10, free from any impurity such as N₂, the nearly closed loop of FT tail-gas can be obtained without the need of inert gas to be vented or removed from the loop.

One advantage of the present process, is that all the undesirable fraction of the distillation unit, i.e. distillation gases that are not recovered as defined fraction, can be recycled to be transformed back into syngas by the means of the plasma reactor.

One other advantage of the present process, is that plasma enables high conversion of the hydrocarbons and CO₂ of the FT tail-gas compare to traditional catalytic reforming. This considerably reduces the remaining unconverted hydrocarbons and CO₂ in the produced syngas. Therefore, the inert component fraction going to the FT process by recycling of FT tail-gas is reduced in comparison to the conventional catalytic reforming. This enhances the FT throughput.

Another key advantage of current invention is electricity is used as an energy source for recycling of FT tail-gas and distillation gas. Considering that green electricity is supplied for this purpose, it can significantly reduce the CO₂ footprint of the process, while enabling higher product yield and higher energy efficiency compared to the conventional reforming process. When the FT tail-gas reforming is performed with traditional reformer like partial oxidation reformer or auto-thermal reformer, usually oxygen is injected in order to convert hydrocarbons to syngas. This in parallel, leads to consumption of H₂ which react with O₂ to form H₂O. This side reaction implies a loss of H₂ and an unwanted formation of H₂O. However, in the present invention FT tail-gas is reformed at relatively low conversion of H₂ into H₂O since O₂ injection is limited. This minimizes the H₂ requirement for overall process. Assuming that H₂ is produced by electrolyser for the fuel plant, current invention saves energy by minimizing the H₂ requirement for the e-fuel production. Therefore, the overall process is energy efficient. Although O₂ supply is proposed as possibility for the regulation of syngas composition in case of very high amounts of hydrocarbons are present, the O₂ demand and therefore H₂ loss in reaction with oxygen will always be lower in current setup compared to conventional catalytic partial oxidation or auto thermal reforming.
Fig. 2 shows a second embodiment, which differs from Fig.1 only in the way of producing the first stream of hot syngas S1a. In this embodiment, the first stream of hot syngas is produced in a co-electrolyser reactor 11a, fed with a stream A20 of CO₂ and a stream A30 of H₂O. Advantageously, the FT tail-gas A70 can also be injected (not shown) into co-electrolyser reactor 11a, which can be combined with classical catalyst-based reformer.

Fig.3 shows a third embodiment, which differs from Fig. 1 in the way of producing the dry syngas S3. The dry syngas is produced from an integrated plasma-based RWGS reactor 110, where plasma-based treatment and RWGS reaction are coupled and occurs in the said unit 110.

The plasma-based RWGS reactor 110 is supplied with a stream A31 of H₂, A20 of CO₂ and streams A70 and A80 from respectively FT reactor 140 and distillation/upgrading unit 150 as recycling gas. Optionally, a regulating stream A10 can be added as reactant (comprising H₂O and/or CO and/or O₂ and or hydrocarbons) to regulate the amount of gases to obtain a combined hot syngas S1 where the amount of remaining CO₂ and hydrocarbons is less than 40 vol %, preferably less than 25 vol %, more preferably less than 15 vol % of the total amount of syngas S1 components.

In a conventional RWGS reactor operating at 1000°C, conversion of CO₂ into CO is driven by the amount of H₂ inside the reactor. A large amount of H₂ is thus needed to have a high conversion of CO₂ and to obtain a syngas comprising H₂ and CO in a molar ratio of H₂/CO from 1.90/1 to 2.10/1 which is needed for the FT reaction. With the embodiment shown in Fig.2, the RWGS reaction occurs in a plasma reactor at a temperature higher than 1250 °C. At this reaction condition the RWGS reaction requires less H₂ content and no catalyst to convert the CO₂. Other reactions also take place in the plasma state such as methane reforming, partial oxidation, etc... One advantage of this plasma-based RWGS reactor 110, is that the amount of H₂ needed for the RWGS reaction, i.e. the conversion of H₂ + CO₂ into H₂ + CO is lower than in a conventional RWGS reaction, leading to a considerable economy in H₂ consumption, therefore a reduction of H₂ production by electrolysis and therefore saving energy. As the syngas will be further used in FT reactor 140, the resulting combined hot syngas S1 is cooled down in a cooling/quenching unit 120, to quench the reaction and to obtain a cooled syngas S2 at the desired H₂/CO ratio and temperature. Quenching/cooling/heat recovery unit 120 may contain heat recovery unit to utilize the sensible heat of the syngas coming from unit 110. This recovered heat can be used to heat up the inlet streams such as A10, A20, A31, A70, A80 and/or for some other purposes such as production of steam for electrolysis or elsewhere. The stream S2 is further sent to a drying unit 130 to form the dry syngas S3. A step of compression (not shown) can be realized after the drying unit 130 if the pressure of stream S2 is not suitable for the FT reactor 140.

The H₂ content of the resulting dry syngas S3, may need to be adjusted to a molar ratio of H₂/CO between 2.1/1 to 2.35/1. This can either be done by feeding H₂ (A31) to unit 110 with H₂ heated at high temperature, or preferably by adding H₂ downstream the reactor 110 directly, or after the quenching/cooling/heat recovery unit 120 in stream S2 and/or S3. By doing so, the H₂ stream can be added at low temperature without the need to heat H₂ at high temperature. This will further improve the energy efficiency of the process. A compression step might be needed in unit 130, if there is difference in pressure compared to the syngas coming from unit 120, and requirement of FT reactor 140. The condensate/steam A40 produced during the cooling and drying process at unit 120 and/or 130, can be recovered and used for the production of H₂ by electrolysis (not shown). The stream exiting the FT reactor 140 is then divided into stream A50 and A70. The higher hydrocarbons stream A50 is sent to upgrading/distillation unit 150 to produce the defined fraction of higher hydrocarbons A60. The FT tail-gas stream A70 and the distillation waste gas stream A80 from unit 150 are reintroduced into the plasma-based RWGS reactor 110 to be recycled.

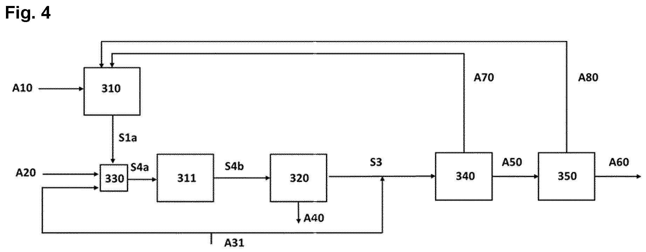

Fig.4 shows another embodiment where the second stream of hot syngas S1a formed in the plasma-based reactor 310 is sent to a mixing unit 330 upstream the RWGS reactor 311. The mixing unit 330 is further fed with CO₂, stream A20 from direct air capture or other sources, and with H₂, stream A31. One advantage of this embodiment is the second stream of hot syngas S1a, exits the plasma-based treatment unit 310 at a temperature higher than 1000° C. This hot syngas S1a is mixed with stream A20 and A31, and exit mixing unit 330 as a third stream of hot syngas S4a. Depending on the temperature and flow of S1a and the desired flow of S4a, an additional heating step can be realized before or in the RWGS reactor 311 in order to meet the heat requirement of RWGS reactor 311. Advantageously, no additional heating step is required if the temperature of the S1a and A20 as well as A31 is sufficient to ensure the heating of S4a at a temperature where it meets the required heat demand of RWGS reactor 311. i.e. temperature above about 900 °C, preferably above about 950°C. S4a is further sent to the RWGS reactor 311 to give the fourth stream of hot syngas S4b. Since S4a enters RWGS reactor 311 at high temperature, the energy needed to heat unit 311 is reduced compare to conventional RWGS reactor. The resulting hot syngas S4b, is further quenched/cooled in a quenching/cooling/heat recovery unit 320 to produce dry syngas S3. A stream A40 of condensate steam can be recovered. Moreover, heat recovery can be used to heat the inlet gases, A20 and A31 for RWGS reactor 311, and the FT tail-gas stream A70 for plasma-based reactor 310 reducing the overall supply of energy. Dry syngas S3 is further sent to the FT unit 340, forming stream A50 of liquid hydrocarbons and FT tail-gas A70. Liquid hydrocarbons A50 are further treated in an upgrading/distillation unit 350, forming liquid fuel A60 and distillation waste gas A80. Stream A70 and A80 are sent back to the plasma-based treatment unit 310. Compression steps (not shown) of streams S1a, S4a, S4b, S3 can be performed to adjust the pressure of the streams if needed. Moreover, a stream of H₂, A31 can be mixed to stream S3 to adjust the H₂/CO ratio of S3.

### Legend:

- 10: Plasma-based reactor
- 11: RWGS reactor
- 11a: Co-electrolyser reactor
- 20: Quenching/cooling/heat recovery unit
- 21: Quenching/cooling/heat recovery unit
- 30: Drying/mixing unit
- 40: FT reactor
- 50: Upgrading/distillation unit
- 110: Plasma-based RWGS reactor unit
- 120: Quenching/cooling/heat recovery unit
- 130: Drying unit
- 310: Plasma-based reactor
- 311: RWGS reactor
- 320: Quenching/cooling/heat recovery/drying unit
- 330: Mixing unit
- 340: FT reactor
- 350: Upgrading/distillation unit
- A10: Regulating stream
- A20: CO₂ stream
- A30: H₂O stream
- A31: H₂ stream
- A40: Steam condensate
- A41: Steam condensate
- A50: Higher hydrocarbons
- A60: Defined hydrocarbon fraction
- A70: FT tail-gas
- A80: Distillation waste gas
- S1a: Second stream of hot syngas
- S1b: First stream of hot syngas
- S2a: Second stream of cooled syngas
- S2b: First stream of cooled syngas
- S1: Combine stream of hot syngas
- S2: Cooled syngas
- S3: Dry syngas
- S4a: Third stream of hot syngas
- S4b: Fourth stream of hot syngas

## Claims

1. A method for producing higher hydrocarbons in a Fischer-Tropsch (FT) reactor by recycling a FT tail-gas comprising:
- forming a dry syngas by
∘ feeding a Reverse Water-Gas Shift (RWGS) reaction with a stream of CO₂ and a stream of H₂ and/or feeding a co-electrolyser reactor with a stream of CO₂ and a stream of H₂O, and
∘ feeding a plasma-based reactor with a FT tail-gas,
- feeding the FT reactor with the dry syngas to form higher hydrocarbons and said FT tail-gas as by-product,
wherein the dry syngas fed to the FT reactor comprises < 5 vol.-% of H₂O, wherein higher hydrocarbons are hydrocarbons having a chain in the range of C6 to C100, and wherein the FT tail-gas comprises hydrocarbons having a carbon chain with up to 5 carbon atoms and unreacted gases.

2. The method according to claim 1, wherein the FT tail-gas is treated in the co-electrolysis.

3. The method according to claim 1 or 2, wherein the higher hydrocarbons are fed to a distillation unit to obtain one or more defined fractions of higher hydrocarbons and a distillation waste fraction; and wherein the distillation waste fraction is treated by said plasma-based treatment.

4. The method according to any of the preceding claims, wherein the dry syngas has a H₂/CO ratio of 1.70/1 to about 2.50/1, preferably of 1.80/1 to 2.30/1, more preferably 1.90/1 to 2.10/1, said H₂/CO ratio being controlled by adding regulating streams comprising CO₂, and/or H₂, and/or H₂O and/or O₂ to the plasma-based treatment of FT tail gas, and/or by controlling the stream of CO₂ and/or the stream of H₂ fed to the RWGS reaction, and/or by controlling the stream of CO₂ and/or H₂O and/or FT tail-gas fed to the co-electrolysis, and/or by mixing an additional stream of H₂ to the dry syngas.

5. The method according to any of the preceding claims, wherein further components within the dry syngas represent < 50 vol.-%, preferably < 35 vol.-%, more preferably < 25 vol.-%, even more preferably < 15 vol.-% of dry syngas.

6. The method according to any of the preceding claims, wherein the dry syngas fed to the FT reactor comprises < 3 vol.-%, preferably < 1 vol.% of H₂O.

7. The method according to any of the preceding claims, wherein the FT reactor is operated at temperatures from 180 °C to 350 °C, preferably from 200 °C to 230 °C and/or at pressures from 5 to 60 barg, preferably from 10 to 50 barg more preferably from 20 to 30 barg, and with cobalt and/or iron as catalyst.

8. The method according to any of the preceding claims, wherein the stream of H₂ is produced by electrolysis and/or the stream of CO₂ is recovered from direct air capture, combustion gases and/or other industrial off-gases, preferably from direct air capture.

9. The method according to any of the preceding claims, wherein the plasma-based treatment is operated with plasma at temperatures higher than 1000 °C, preferably higher than 1250 °C, more preferably higher than 1500 °C, even more preferably higher than 2000° C and at pressure lower than 45 barg, preferably lower than 20 barg, more preferably lower than 5 barg.

10. The method according to any of the preceding claims, wherein the RWGS reaction is done in a RWGS reactor to produce a first stream of hot syngas, the first stream of hot syngas being treated in a first quenching/cooling/heat recovery unit to form a first stream of cooled syngas; and wherein, the plasma-based treatment of FT tail-gas is done in a plasma-based reactor to produce a second stream of hot syngas, the second stream of hot syngas being treated in a second quenching/cooling/heat recovery unit to form a second stream of cooled syngas; and wherein the first and second streams of cooled syngas are dried and preferably mixed in a drying/mixing unit to obtain the dry syngas.

11. The method according to claims 1 to 9, wherein the co-electrolysis reaction is done in a co-electrolysis reactor to produce a first stream of hot syngas, the first stream of hot syngas being treated in a first quenching/cooling/heat recovery unit to form a first stream of cooled syngas; and wherein, the plasma-based treatment of FT tail-gas is done in a plasma-based reactor to produce a second stream of hot syngas, the second stream of hot syngas being treated in a second quenching/cooling/heat recovery unit to form a second stream of cooled syngas; and wherein the first and second streams of cooled syngas are dried and preferably mixed in a drying/mixing unit to obtain the dry syngas.

12. The method according to claim 9 to 11, wherein a stream of H₂ is mixed with the second stream of hot syngas, and/or is mixed with the first stream of cooled syngas or is mixed with the dry syngas.

13. The method according to any of claims 1 to 9, wherein the RWGS reaction and the plasma-based treatment are done in a plasma-based RWGS reactor fed with a stream of H₂, and/or a stream of CO₂ and/or a stream of FT tail-gas and/or a stream of distillation waste gas to form a combined stream of hot syngas; wherein the combined stream of hot syngas is treated in a combined quenching/cooling/heat recovery unit to obtain the cooled syngas.

14. The method according to claim 13, wherein a stream of H₂ is added in the combined stream of hot syngas to control the H₂/CO ratio.

15. The method according to any of claims 1 to 9, wherein the plasma-based treatment of FT tail-gas is done in a plasma-based reactor to produce a second stream of hot syngas, the second stream of hot syngas being mixed with streams of CO₂ and H₂ to form a third stream of hot syngas; and wherein the third stream of hot syngas is treated in a RWGS reactor to form a fourth stream of hot syngas; and wherein the fourth stream of hot syngas is cooled and dried in a quenching/cooling/heat recovery/drying unit to form a stream of dry syngas.

16. The method according to claim 15, wherein a stream of H₂ is added to the stream of dry syngas.

17. The method according to any of preceding claims, wherein the heat from the hot syngas is recovered in a heat recovery unit during cooling, said recovered heat is further utilized to heat the inlet FT tail-gas of the plasma-based reactor and/or inlet gas for RWGS reactor and/or the dry syngas.

18. The method according to any of the preceding claims, wherein the first and/or second stream of hot syngas, and/or the combined stream of hot syngas, and/or the first and/or second stream of cooled syngas, and/or the stream of cooled syngas, and/or the stream of dry syngas is compressed in a compression unit, preferably the first and/or second stream of cooled syngas and/or the stream of cooled syngas, more preferably, the dry syngas is compressed in a compression unit.

19. The method according to any of the preceding claims, wherein heat generated during quenching is utilized for production of steam.

20. The method according to any of the preceding claims, wherein steam generated from the heat recovery unit is utilized in the co-electrolysis for production of hydrogen and/or syngas.

21. The method according to any of the preceding claims, wherein the plasma-based treatment unit comprises one or more plasma torches, preferably electrode-based plasma torches or electrodeless plasma torches, selected from inductively ignited plasma torches, microwave plasma torches or a combination thereof.

22. The method according to claim 21, wherein transferred and/or non-transferred arcs are employed to facilitate the plasma-based treatment.

23. The method according to claim 21, wherein the one or more plasma torches include direct current plasma torches and/or alternating current plasma torches and/or 3-phase alternating current plasma torches, wherein said plasma torches preferably have a total electric power of 1 to 20 MW, more preferably of 3 to 15 MW, even more preferably of 5 to 10 MW.

## Patentansprüche

1. Verfahren zur Herstellung von höheren Kohlenwasserstoffen in einem Fischer-Tropsch-Reaktor (FT-Reaktor) durch Recyceln eines FT-Endgases, umfassend:
- Bilden eines trockenen Synthesegases durch
∘ Versorgen einer umgekehrten Wasser-Gas-Shift-Reaktion (RWGS-Reaktion) mit einem Strom von CO₂ und einem Strom von H₂ und/oder Versorgen eines Co-Elektrolyseur-Reaktors mit einem Strom von CO₂ und einem Strom von H₂O und
∘ Versorgen eines auf Plasma basierenden Reaktors mit einem FT-Endgas,
- Versorgen des FT-Reaktors mit dem trockenen Synthesegas, um höhere Kohlenwasserstoffe und das FT-Endgas als Nebenprodukt zu bilden,
wobei das dem FT-Reaktor zugeführte trockene Synthesegas < 5 Vol.-% H₂O umfasst, wobei höhere Kohlenwasserstoffe Kohlenwasserstoffe mit einer Kette im Bereich von C6 bis C100 sind und wobei das FT-Endgas Kohlenwasserstoffe mit einer Kohlenstoffkette mit bis zu 5 Kohlenstoffatomen und nicht umgesetzten Gasen umfasst.

2. Verfahren nach Anspruch 1, wobei das FT-Endgas in der Co-Elektrolyse behandelt wird.

3. Verfahren nach Anspruch 1 oder 2, wobei die höheren Kohlenwasserstoffe einer Destillationseinheit zugeführt werden, um eine oder mehr definierte Fraktionen von höheren Kohlenwasserstoffen und eine Destillationsabfallfraktion zu erhalten; und wobei die Destillationsabfallfraktion durch die auf Plasma basierende Behandlung behandelt wird.

4. Verfahren nach einem der vorhergehenden Ansprüche, wobei das trockene Synthesegas ein H₂/CO-Verhältnis von 1,70/1 bis etwa 2,50/1, vorzugsweise von 1,80/1 bis 2,30/1, stärker bevorzugt 1,90/1 bis 2,10/1 aufweist, wobei das H₂/CO-Verhältnis durch Zugabe von regulierenden Strömen, umfassend CO₂ und/oder H₂ und/oder H₂O und/oder O₂ zu der auf Plasma basierenden Behandlung von FT-Endgas gesteuert wird, und/oder durch Steuern des Stroms von CO₂ und/oder des Stroms von H₂, der der RWGS-Reaktion zugeführt wird, und/oder durch Steuern des Stroms von CO₂ und/oder H₂O und/oder FT-Endgas, der der Co-Elektrolyse zugeführt wird, und/oder durch Mischen eines zusätzlichen Stroms von H₂ zu dem trockenen Synthesegas.

5. Verfahren nach einem der vorhergehenden Ansprüche, wobei weitere Komponenten innerhalb des trockenen Synthesegases < 50 Vol.-%, vorzugsweise < 35 Vol.-%, stärker bevorzugt < 25 Vol.-%, sogar noch stärker bevorzugt < 15 Vol.-% von trockenem Synthesegas darstellen.

6. Verfahren nach einem der vorhergehenden Ansprüche, wobei das dem FT-Reaktor zugeführte trockene Synthesegas < 3 Vol.-%, vorzugsweise < 1 Vol.-% H₂O umfasst.

7. Verfahren nach einem der vorhergehenden Ansprüche, wobei der FT-Reaktor bei Temperaturen von 180 °C bis 350 °C, vorzugsweise von 200 °C bis 230 °C, und/oder bei Drücken von 5 bis 60 barg, vorzugsweise von 10 bis 50 barg, stärker bevorzugt von 20 bis 30 barg, und mit Kobalt und/oder Eisen als Katalysator betrieben wird.

8. Verfahren nach einem der vorhergehenden Ansprüche, wobei der Strom von H₂ durch Elektrolyse hergestellt wird und/oder der Strom von CO₂ aus direkter Gewinnung aus der Luft, Verbrennungsabgasen und/oder anderen industriellen Abgasen, vorzugsweise aus direkter Gewinnung aus der Luft, hergestellt wird.

9. Verfahren nach einem der vorhergehenden Ansprüche, wobei die auf Plasma basierende Behandlung mit Plasma bei Temperaturen höher als 1000 °C, vorzugsweise höher als 1250 °C, stärker bevorzugt höher als 1500 °C, sogar noch stärker bevorzugt höher als 2000 °C, und einem Druck von weniger als 45 barg, vorzugsweise weniger als 20 barg, stärker bevorzugt weniger als 5 barg betrieben wird.

10. Verfahren nach einem der vorhergehenden Ansprüche, wobei die RWGS-Reaktion in einem RWGS-Reaktor erfolgt, um einen ersten Strom von heißem Synthesegas herzustellen, wobei der erste Strom von heißem Synthesegas in einer ersten Quenching-/Kühl-/Wärmerückgewinnungseinheit behandelt wird, um einen ersten Strom von gekühltem Synthesegas zu bilden; und wobei die auf Plasma basierende Behandlung von FT-Endgas in einem auf Plasma basierenden Reaktor erfolgt, um einen zweiten Strom von heißem Synthesegas herzustellen, wobei der zweite Strom von heißem Synthesegas in einer zweiten Quenching-/Kühl-/Wärmerückgewinnungseinheit behandelt wird, um einen zweiten Strom von gekühltem Synthesegas zu bilden; und wobei der erste und der zweite Strom von gekühltem Synthesegas getrocknet und vorzugsweise in einer Trocknungs-/Mischeinheit gemischt werden, um das trockene Synthesegas zu erhalten.

11. Verfahren nach den Ansprüchen 1 bis 9, wobei die Co-Elektrolysereaktion in einem Co-Elektrolysereaktor erfolgt, um einen ersten Strom von heißem Synthesegas herzustellen, wobei der erste Strom von heißem Synthesegas in einer ersten Quenching-/Kühl-/Wärmerückgewinnungseinheit behandelt wird, um einen ersten Strom von gekühltem Synthesegas zu bilden; und wobei die auf Plasma basierende Behandlung von FT-Endgas in einem auf Plasma basierenden Reaktor erfolgt, um einen zweiten Strom von heißem Synthesegas herzustellen, wobei der zweite Strom von heißem Synthesegas in einer zweiten Quenching-/Kühl-/Wärmerückgewinnungseinheit behandelt wird, um einen zweiten Strom von gekühltem Synthesegas zu bilden; und wobei der erste und der zweite Strom von gekühltem Synthesegas getrocknet und vorzugsweise in einer Trocknungs-/Mischeinheit gemischt werden, um das trockene Synthesegas zu erhalten.

12. Verfahren nach den Ansprüchen 9 bis 11, wobei ein Strom von H₂ mit dem zweiten Strom von heißem Synthesegas gemischt wird und/oder mit dem ersten Strom von gekühltem Synthesegas gemischt wird oder mit dem trockenen Synthesegas gemischt wird.

13. Verfahren nach einem der Ansprüche 1 bis 9, wobei die RWGS-Reaktion und die auf Plasma basierende Behandlung in einem auf Plasma basierenden RWGS-Reaktor erfolgen, der mit einem Strom von H₂ und/oder einem Strom von CO₂ und/oder einem Strom von FT-Endgas und/oder einem Strom von Destillationsabfallgas versorgt wird, um einen kombinierten Strom von heißem Synthesegas zu bilden; wobei der kombinierte Strom von heißem Synthesegas in einer kombinierten Quenching-/Kühl-/Wärmerückgewinnungseinheit behandelt wird, um das gekühlte Synthesegas zu erhalten.

14. Verfahren nach Anspruch 13, wobei ein Strom von H₂ in den kombinierten Strom von heißem Synthesegas zugegeben wird, um das H₂/CO-Verhältnis zu steuern.

15. Verfahren nach einem der Ansprüche 1 bis 9, wobei die auf Plasma basierende Behandlung von FT-Endgas in einem auf Plasma basierenden Reaktor erfolgt, um einen zweiten Strom von heißem Synthesegas herzustellen, der zweite Strom von heißem Synthesegas mit Strömen von CO₂ und H₂ gemischt wird, um einen dritten Strom von heißem Synthesegas zu bilden; und wobei der dritte Strom von heißem Synthesegas in einem RWGS-Reaktor behandelt wird, um einen vierten Strom von heißem Synthesegas zu bilden; und wobei der vierte Strom von heißem Synthesegas in einer Quenching-/Kühl-/Wärmerückgewinnungs-/Trocknungseinheit gekühlt und getrocknet wird, um einen Strom von trockenem Synthesegas zu bilden.

16. Verfahren nach Anspruch 15, wobei ein Strom von H₂ dem Strom von trockenem Synthesegas zugegeben wird.

17. Verfahren nach einem der vorhergehenden Ansprüche, wobei die Wärme von dem heißen Synthesegas während des Kühlens in einer Wärmerückgewinnungseinheit zurückgewonnen wird, die zurückgewonnene Wärme weiter genutzt wird, um das FT-Einlassendgas des auf Plasma basierenden Reaktors und/oder das Einlassgas für den RWGS-Reaktor und/oder das trockene Synthesegas zu erwärmen.

18. Verfahren nach einem der vorhergehenden Ansprüche, wobei der erste und/oder zweite Strom von heißem Synthesegas und/oder der kombinierte Strom von heißem Synthesegas und/oder der erste und/oder zweite Strom von gekühltem Synthesegas und/oder der Strom von gekühltem Synthesegas und/oder der Strom von trockenem Synthesegas in einer Verdichtungseinheit verdichtet wird, vorzugsweise der erste und/oder zweite Strom von gekühltem Synthesegas und/oder der Strom von gekühltem Synthesegas, stärker bevorzugt das trockene Synthesegas, in einer Verdichtungseinheit verdichtet wird.

19. Verfahren nach einem der vorhergehenden Ansprüche, wobei während des Quenching erzeugte Wärme zur Erzeugung von Dampf genutzt wird.

20. Verfahren nach einem der vorhergehenden Ansprüche, wobei von der Wärmerückgewinnungseinheit erzeugter Dampf bei der Co-Elektrolyse zur Herstellung von Wasserstoff und/oder Synthesegas genutzt wird.

21. Verfahren nach einem der vorhergehenden Ansprüche, wobei die auf Plasma basierende Behandlungseinheit eine oder mehrere Plasmabrenner, vorzugsweise auf Elektroden basierende Plasmabrenner oder elektrodenlose Plasmabrenner umfasst, ausgewählt aus induktiv gezündeten Plasmabrennern, Mikrowellenplasmabrennern oder einer Kombination davon.

22. Verfahren nach Anspruch 21, wobei übertragene und/oder nicht übertragene Lichtbögen eingesetzt werden, um die auf Plasma basierende Behandlung zu vereinfachen.

23. Verfahren nach Anspruch 21, wobei der eine oder die mehreren Plasmabrenner Gleichstrom-Plasmabrenner und/oder Wechselstrom-Plasmabrenner und oder 3-Phasen-Wechselstrom-Plasmabrenner einschließen, wobei die Plasmabrenner vorzugsweise eine elektrische Gesamtleistung von 1 bis 20 MW, stärker bevorzugt von 3 bis 15 MW, sogar noch stärker bevorzugt von 5 bis 10 MW aufweisen.

## Revendications

1. Procédé de production d'hydrocarbures supérieurs dans un réacteur de Fischer-Tropsch (FT) par recyclage d'un gaz de queue de FT comprenant :
- le fait de former un gaz de synthèse sec par
∘ une réaction de gaz à l'eau inverse (RWGS) alimenté avec un courant de CO₂ et un courant de H₂ et/ou par un réacteur de co-électrolyse alimenté avec un courant de CO₂ et un courant de H₂O, et
∘ un réacteur à plasma alimenté avec du gaz de queue de FT
- le fait d'alimenter le réacteur de FT avec le gaz de synthèse sec pour former des hydrocarbures supérieurs et le gaz de queue de FT en tant que sous-produit,
dans lequel le gaz de synthèse sec alimenté au réacteur de FT comprend < 5% en volume de H₂O, dans lequel les hydrocarbures supérieurs sont des hydrocarbures ayant une chaîne dans la plage de C6 à C100, et dans lequel le gaz de queue de FT comprend des hydrocarbures ayant une chaîne carbonée avec jusqu'à 5 atomes de carbone et des gaz non réagis.

2. Procédé selon la revendication 1, dans lequel le gaz de queue de FT est traité dans la co-électrolyse.

3. Procédé selon la revendication 1 ou 2, dans lequel les hydrocarbures supérieurs sont introduits dans une unité de distillation pour obtenir une ou plusieurs fractions définies d'hydrocarbures supérieurs et une fraction de résidus de distillation ; et dans lequel la fraction de résidus de distillation est traitée par ledit traitement au plasma.

4. Procédé selon l'une quelconque des revendications précédentes, dans lequel le gaz de synthèse sec présente un rapport H₂/CO de 1,70/1 à environ 2,50/1, de préférence de 1,80/1 à 2,30/1, plus préférentiellement de 1,90/1 à 2,10/1, ledit rapport H₂/CO étant régulé par ajout de courants régulateurs comprenant CO₂, et/ou H₂, et/ou H₂O et/ou O₂ au traitement au plasma du gaz de queue de FT, et/ou par régulation du courant de CO₂ et/ou du courant de H₂ introduits dans la réaction RWGS, et/ou par régulation du courant de CO₂ et/ou de H₂O et/ou du gaz de queue de FT introduits dans la co-électrolyse, et/ou par mélange d'un courant supplémentaire de H₂ au gaz de synthèse sec.

5. Procédé selon l'une quelconque des revendications précédentes, dans lequel d'autres composants qui se trouvent dans le gaz de synthèse sec représentent < 50 % en volume, de préférence < 35 % en volume, plus préférentiellement < 25 % en volume, encore plus préférentiellement < 15 % en volume du gaz de synthèse sec.

6. Procédé selon l'une quelconque des revendications précédentes, dans lequel le gaz de synthèse sec introduit dans le réacteur de FT comprend < 3 % en volume, préférentiellement < 1 % en volume de H₂O.

7. Procédé selon l'une quelconque des revendications précédentes, dans lequel on fait fonctionner le réacteur de FT à des températures de 180 °C à 350 °C, de préférence de 200 °C à 230 °C et/ou à des pressions de 5 à 60 barg, de préférence de 10 à 50 barg, plus préférentiellement de 20 à 30 barg, et avec du cobalt et/ou du fer comme catalyseur.

8. Procédé selon l'une quelconque des revendications précédentes, dans lequel le courant de H₂ est produit par électrolyse et/ou le courant de CO₂ est récupéré via une capture directe à partir de l'air, des gaz de combustion et/ou d'autres effluents gazeux industriels, de préférence via une capture directe à partir de l'air.

9. Procédé selon l'une quelconque des revendications précédentes, dans lequel le traitement au plasma est mis en œuvre avec du plasma à des températures supérieures à 1000 °C, de préférence supérieures à 1250 °C, plus préférentiellement supérieures à 1500 °C, encore plus préférentiellement supérieures à 2000 °C, et à une pression inférieure à 45 barg, de préférence inférieure à 20 barg, plus préférentiellement inférieure à 5 barg.

10. Procédé selon l'une quelconque des revendications précédentes, dans lequel la réaction RWGS est effectuée dans un réacteur RWGS pour produire un premier courant de gaz de synthèse chaud, le premier courant de gaz de synthèse chaud étant traité dans une première unité de trempe/refroidissement/récupération de chaleur pour former un premier courant de gaz de synthèse refroidi ; et dans lequel le traitement au plasma du gaz de queue de FT est effectué dans un réacteur à plasma pour produire un deuxième courant de gaz de synthèse chaud, le deuxième courant de gaz de synthèse chaud étant traité dans une deuxième unité de trempe/refroidissement/récupération de chaleur pour former un deuxième courant de gaz de synthèse refroidi ; et dans lequel le premier et le deuxième courant de gaz de synthèse refroidi sont séchés et de préférence mélangés dans une unité de séchage/mélangeage pour obtenir le gaz de synthèse sec.

11. Procédé selon les revendications 1 à 9, dans lequel la réaction de co-électrolyse est effectuée dans un réacteur de co-électrolyse pour produire un premier courant de gaz de synthèse chaud, le premier courant de gaz de synthèse chaud étant traité dans une première unité de trempe/refroidissement/récupération de chaleur pour former un premier courant de gaz de synthèse refroidi ; et dans lequel le traitement au plasma du gaz de queue de FT est effectué dans un réacteur à plasma pour produire un deuxième courant de gaz de synthèse chaud, le deuxième courant de gaz de synthèse chaud étant traité dans une deuxième unité de trempe/refroidissement/récupération de chaleur pour former un deuxième courant de gaz de synthèse refroidi ; et dans lequel le premier et le deuxième courant de gaz de synthèse refroidi sont séchés et de préférence mélangés dans une unité de séchage/mélangeage pour obtenir le gaz de synthèse sec.

12. Procédé selon les revendications 9 à 11, dans lequel un courant de H₂ est mélangé avec le deuxième courant de gaz de synthèse chaud, et/ou est mélangé avec le premier courant de gaz de synthèse refroidi ou est mélangé avec le gaz de synthèse sec.

13. Procédé selon l'une quelconque des revendications 1 à 9, dans lequel la réaction RWGS et le traitement au plasma sont effectués dans un réacteur RWGS à plasma alimenté avec un courant de H₂, et/ou un courant de CO₂ et/ou un courant de gaz de queue de FT et/ou un courant de gaz de résidus de distillation pour former un courant combiné de gaz de synthèse chaud ; dans lequel le courant combiné de gaz de synthèse chaud est traité dans une unité combinée de trempe/refroidissement/récupération de chaleur pour obtenir le gaz de synthèse refroidi.

14. Procédé selon la revendication 13, dans lequel un courant de H₂ est ajouté dans le courant combiné de gaz de synthèse chaud pour réguler le rapport H₂/CO.

15. Procédé selon l'une quelconque des revendications 1 à 9, dans lequel le traitement au plasma du gaz de queue de FT est effectué dans un réacteur à plasma pour produire un deuxième courant de gaz de synthèse chaud, le deuxième courant de gaz de synthèse chaud étant mélangé avec des courants de CO₂ et de H₂ pour former un troisième courant de gaz de synthèse chaud ; et dans lequel le troisième courant de gaz de synthèse chaud est traité dans un réacteur RWGS pour former un quatrième courant de gaz de synthèse chaud ; et dans lequel le quatrième courant de gaz de synthèse chaud est refroidi et séché dans une unité de trempe/refroidissement/récupération de chaleur/séchage pour former un courant de gaz de synthèse sec.

16. Procédé selon la revendication 15, dans lequel un courant de H₂ est ajouté au courant de gaz de synthèse sec.

17. Procédé selon l'une quelconque des revendications précédentes, dans lequel la chaleur du gaz de synthèse chaud est récupérée dans une unité de récupération de chaleur pendant le refroidissement, ladite chaleur récupérée est ensuite utilisée pour chauffer le gaz de queue de FT d'admission du réacteur à plasma et/ou le gaz d'admission pour le réacteur RWGS et/ou le gaz de synthèse sec.

18. Procédé selon l'une quelconque des revendications précédentes, dans lequel le premier et/ou deuxième courant de gaz de synthèse chaud, et/ou le courant combiné de gaz de synthèse chaud, et/ou le premier et/ou deuxième courant de gaz de synthèse refroidi, et/ou le courant de gaz de synthèse refroidi, et/ou le courant de gaz de synthèse sec est compressé dans une unité de compression, de préférence le premier et/ou deuxième courant de gaz de synthèse refroidi et/ou le courant de gaz de synthèse refroidi, plus préférentiellement le gaz de synthèse sec est compressé dans une unité de compression.

19. Procédé selon l'une quelconque des revendications précédentes, dans lequel la chaleur générée pendant la trempe est utilisée pour la production de vapeur.

20. Procédé selon l'une quelconque des revendications précédentes, dans lequel la vapeur générée par l'unité de récupération de chaleur est utilisée dans la co-électrolyse pour la production d'hydrogène et/ou de gaz de synthèse.

21. Procédé selon l'une quelconque des revendications précédentes, dans lequel l'unité de traitement au plasma comprend une ou plusieurs torches à plasma, de préférence des torches à plasma à électrodes ou des torches à plasma sans électrodes, choisies parmi les torches à plasma allumées par induction, les torches à plasma micro-ondes ou une combinaison de celles-ci.

22. Procédé selon la revendication 21, dans lequel des arcs transférés et/ou non transférés sont employés pour faciliter le traitement au plasma.

23. Procédé selon la revendication 21, dans lequel la ou les torches à plasma comprennent des torches à plasma à courant continu et/ou des torches à plasma à courant alternatif et/ou des torches à plasma à courant alternatif triphasé, dans lequel lesdites torches à plasma ont de préférence une puissance électrique totale de 1 à 20 MW, plus préférentiellement de 3 à 15 MW, encore plus préférentiellement de 5 à 10 MW.
